# FASCICULE DE BREVET EUROPEEN

(11) **EP 0 647 619 B1**
(45) Date de publication et mention de la délivrance du brevet: **20.05.1998**
(21) Numéro de dépôt: 94420261.3
(22) Date de dépôt: 28.09.1994
(51) Int. Cl.: C07C 255/07, C07F 9/50, C07C 253/10, C07C 253/30

(54) **Procédé d'isomérisation du méthyl-2 butène-3 nitrile**
Verfahren zur Isomerisierung von 2-Methyl-3-butennitril
Process for the preparation of 2-methyl-3-butene nitrile

(30) Priorité: 08.10.1993 FR 9312320
(43) Date de publication de la demande: 12.04.1995
(73) Titulaire: RHODIA FIBER & RESIN INTERMEDIATES, 92408 Courbevoie Cédex (FR)
(72) Inventeur: Huser, Marc, F-69100 Villeurbanne (FR); Perron, Robert, F-69390 Charly (FR)
(74) Mandataire: Vignally, Noel

(56) Documents cités:
- FR-A- 2 033 107
- FR-A- 2 073 605
- FR-A- 2 136 815
- FR-A- 2 338 253
- US-A- 3 536 748
- US-A- 3 637 481

## Description

La présente invention a trait à un procédé d'isomérisation du méthyl-2 butène-3 nitrile en pentène-nitrile linéaire, en particulier en pentène-3 nitrile.

Lors de l'hydrocyanation du butadiène, en présence d'un catalyseur à base de nickel et d'un catalyseur constitué par un phosphite d'aryle, telle que décrite dans le brevet US 3 496 215, il se forme, avec le pentène-3 nitrile qui est le composé visé, une proportion importante de méthyl-2 butène-3 nitrile.

Ce dernier composé n'est pas utilisable directement pour la préparation d'adiponitrile, l'un des composés de base pour la préparation du polyamide 66.

Or les quantités de ce sous-produit sont si importantes, compte-tenu des tonnages énormes d'adiponitrile produits, qu'il est impensable de les détruire.

Aussi a-t-on depuis longtemps cherché à isomériser le méthyl-2 butène-3 nitrile en pentène-3 nitrile, intermédiaire de l'adiponitrile.

Ainsi a-t-il été proposé dans le brevet US 3 536 748 de transformer le méthyl-2 butène-3 nitrile en pentène nitrile linéaire, par mise en contact avec un catalyseur à base de nickel au degré d'oxydation 0, tel qu'un tétrakis(phosphite d'alkyle ou d'aryle) nickel, à une température de 10 à 200°C.

Lorsque l'on étudie les résultats obtenus avec ce procédé d'isomérisation, on constate que le pourcentage de pentène-nitrile linéaire obtenu est très faible, généralement de 0,4 % à environ 14 % selon les exemples du brevet, alors que la réaction dure plusieurs heures.

Le brevet US 3 676 481 décrit une amélioration du procédé d'isomérisation précédent, par l'utilisation d'un promoteur du catalyseur au nickel, constitué soit par un dérivé du bore, soit par un sel de métal appartenant aux groupes Ib, IIb, IIIa, IIIb, IVa, IVb, Vb,VIb, VIIb et VIIII, notamment les halogénures de ces métaux. Comme précédemment, la réaction a lieu en phase organique homogène, ce qui rend difficile la séparation des produits de la réaction et du catalyseur, dans le cadre du recyclage, toujours souhaitable, du catalyseur.

Le brevet français FR-A-2 033 107 décrit un procédé de préparation de nitriles insaturés à partir de butadiène et d'acide cyanhydrique, catalysé par un complexe de nickel de bis(diphényl phosphine) alcane. Le procédé est conduit dans un hydrocarbure aromatique. Les rendements en pentène nitrile sont généralement supérieurs à 90 %.

Le brevet français FR-A-2 073 605 décrit un procédé d'isomérisation du méthyl-2 butène-3 nitrile en pentène-3 nitrile, par chauffage en l'absence de solvant et en présence d'un complexe de nickel de bis(diphényl phosphine)alcane, à une température de 50°C à 150°C, sous pression normale et pendant une durée de 15 à 150 minutes. Les rendements en pentène-3 nitrile varient selon les exemples de 39,7 % à 79,5 %. Ils sont inférieurs aux rendements obtenus avec le même catalyseur pour l'hydrocyanation du butadiène.

Le brevet FR-A-2 338 253 décrit l'hydrocyanation d'un composé organique insaturé comportant au moins une double liaison éthylénique, par réaction du composé insaturé avec l'acide cyanhydrique en présence d'une solution aqueuse d'au moins une triarylphosphine portant sur les cycles aryles un ou plusieurs substituants sulfonate. Le substrat le plus exemplifié est le pentène-3 nitrile.

La présente invention apporte une solution simple et pratique à l'isomérisation du méthyl-2 butène-3 nitrile en pentène nitrile linéaire et plus particulièrement en pentène-3 nitrile.

Plus précisément, elle consiste en un procédé d'isomération du méthyl-2 butène-3 nitrile en un pentène-nitrile linéaire, caractérisé en ce que la réaction est effectuée en présence d'une solution aqueuse :
- d'au moins une phosphine sulfonée de formule générale (I) 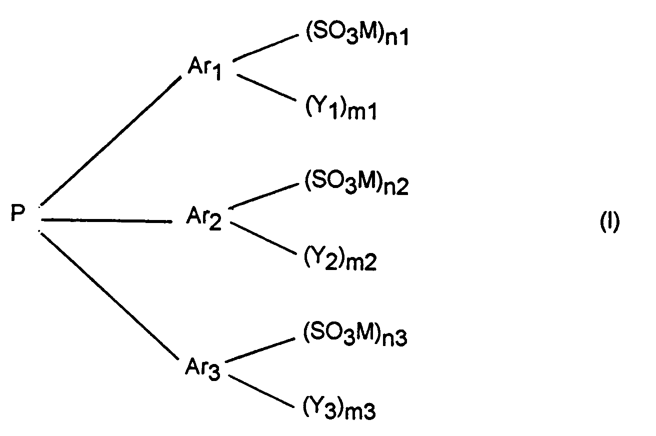 dans laquelle :
   * Ar₁, Ar₂ et Ar₃, identiques ou différents, représentent des groupements aryles
   * Y₁, Y₂ et Y₃, identiques ou différents, représentent
      . un radical alkyle ayant 1 à 4 atomes de carbone,
      . un radical alcoxy ayant 1 à 4 atomes de carbone,
      . un atome d'halogène,
      . un radical CN,
      . un radical NO₂
      . un radical OH,
      . un radical NR₁R₂, dans la formule duquel R₁ et R₂, identiques ou différents, représentent un radical alkyle ayant 1 à 4 atomes de carbone,
   * M est un reste cationique minéral ou organique choisi, de manière à ce que le composé de formule (I) soit soluble dans l'eau, dans le groupe comprenant :
      . H⁺,
      . les cations dérivés des métaux alcalins ou alcalino-terreux,
      . N(R₃R₄R₅R₆)⁺ avec R₃,R₄,R₅ et R₆, identiques ou différents, représentant un radical alkyle ayant 1 à 4 atomes de carbone ou un atome d'hydrogène,
      . les autres cations dérivés des métaux dont les sels de l'acide benzènesulfonique sont solubles dans l'eau,
   * m₁, m₂ et m₃ sont des nombres entiers, identiques ou différents, de 0 à 5,
   * n₁, n₂ et n₃ sont des nombres entiers, identiques ou différents, de 0 à 3, l'un d'entre eux au moins étant égal ou supérieur à 1
- et d'au moins un composé d'un métal de transition.

Comme exemples de métaux dont les sels de l'acide benzènesulfonique sont solubles dans l'eau, on peut citer le plomb, le zinc et l'étain.

Par l'expression soluble dans l'eau, on entend dans le présent texte un composé soluble à au moins 0,01 g par litre d'eau.

Parmi les phosphines de formule (I), on préfère celles pour lesquelles :
- Ar₁, Ar₂ et Ar₃ sont des groupements phényle,
- Y₁, Y₂ et Y₃ représentent des groupements choisis parmi
   . les radicaux alkyle ayant de 1 à 2 atomes de carbone,
   . les radicaux alkoxy ayant de 1 à 2 atomes de carbone,
- M représente un cation choisi parmi le groupe comprenant
   . H⁺
   . les cations dérivés de Na, K, Ca, Ba
   . NH₄+
   . les cations tétraméthylammonium, tétraéthylammonium, tétrapropylammonium, tétrabutylammonium
- m₁, m₂ et m₃ sont des nombres entiers de 0 à 3
- n₁, n₂ et n₃ sont des nombres entiers de 0 à 3, l'un au moins étant également supérieur à 1.

Parmi ces phosphines, les plus particulièrement préférées sont les sels de sodium, de potassium, de calcium, de baryum, d'ammonium, de tétraméthylammonium et de tétraéthylammonium, des mono(sulfophényl) diphényl-phosphine, di(sulfophényl) phényl-phosphine et tri(sulfophényl)-phosphine dans les formules desquelles les groupements SO₃ sont de préférence en position méta.

On peut citer comme autres exemples de phosphines de formule (I) pouvant être utilisées selon le procédé de l'invention, les sels alcalins ou alcalino-terreux, les sels d'ammonium, les sels d'ammonium quaternaire des (sulfo-3 méthyl-4 phényl) di (méthyl-4 phényl)-phosphine ; (sulfo-3 méthoxy-4 phényl) di(méthoxy-4 phényl)-phosphine ; (sulfo-3 chloro-4 phényl) di(chloro-4 phényl)-phosphine ; di(sulfo-3 phényl) phényl-phosphine ; di(sulfo-4 phényl) phényl-phosphine ; di(sulfo-3 méthyl-4 phényl) (méthyl-4 phényl)-phosphine ; di(sulfo-3 méthoxy-4 phényl) (méthoxy-4 phényl) phosphine ; di(sulfo-3 chloro-4 phényl) (chloro-4 phényl) phosphine ; tri(sulfo-3 phényl)-phosphine ; tri(sulfo-4 phényl)-phosphine ; (tri(sulfo--3 méthyl-4 phényl)-phosphine ; tri(sulfo-3, méthoxy-4 phényl)-phosphine ; tri(sulfo-3 chloro-4 phényl)-phosphine ; (sulfo-2 méthyl-4 phényl) (sulfo-3, méthyl-4 phényl) (disulfo-3,5 méthyl-4 phényl)-phosphine ; (sulfo-3 phényl) (sulfo-3 chloro-4 phényl) (disulfo-3,5 chloro-4 phényl)-phosphine.

On peut bien évidemment utiliser un mélange de ces phosphines. En particulier, on peut utiliser un mélange de phosphines mono-, di- et tri-métasulfonées.

On utilise de préférence comme composés des métaux de transition des composés du nickel, du palladium et du fer. On utilise des composés solubles dans l'eau ou capables de passer en solution dans les conditions de la réaction. Le reste lié au métal n'est pas critique, dès l'instant qu'il satisfait à ces conditions.

Parmi les composés précités, les composés les plus préférés sont ceux du nickel. On peut citer à titre d'exemples non limitatifs :
- les composés dans lesquel le nickel est au degré d'oxydation zéro comme le tétracyanonickelate de potassium K₄ (NiCN₄), le bis (acrylonitrile) nickel, le bis (cyclooctadiène 1,5)₂ nickel et les dérivés contenant des ligands du groupe Va comme le tétrakis (triphényl-phosphine) nickel (dans ce dernier cas le composé peut être dissous dans un solvant non miscible à l'eau comme le toluène, puis une solution aqueuse de phosphine sulfonée extrait une partie du nickel en développant une coloration rouge dans la solution aqueuse qui décante);
- les composés du nickel à un degré d'oxydation supérieur à 0, comme les carboxylates (notamment l'acétate), carbonate, bicarbonate, borate, bromure, chlorure, citrate, thiocyanate, cyanure, formiate, hydroxyde, hydrophosphite, phosphite, phosphate et dérivés, iodure, nitrate, sulfate, sulfite, aryl- et alkyl-sulfonates.

Il n'est pas nécessaire que le composé du nickel soit lui même soluble dans l'eau. Par exemple, le cyanure de nickel peu soluble dans l'eau se dissout très bien dans une solution aqueuse de phosphine.

Quand le composé du nickel utilisé correspond à un état d'oxydation du nickel supérieur à 0, on ajoute au milieu réactionnel un réducteur du nickel réagissant préférentiellement avec le nickel dans les conditions de la réaction. Ce réducteur peut être organique ou minéral. On peut citer comme exemples non limitatifs le BH₄Na, la poudre de Zn, le magnésium, le BH₄K et les borohydrures de préférence solubles dans l'eau.

Ce réducteur est ajouté en quantité telle que le nombre d'équivalents d'oxydo-réduction est compris entre 1 et 10. Des valeurs inférieures à 1 et supérieures à 10 ne sont toutefois pas exclues.

Lorsque le composé du nickel utilisé correspond à l'état d'oxydation 0 du nickel, on peut également ajouter un réducteur du type de ceux précités, mais cet ajout n'est pas impératif.

Lorsqu'on utilise un composé du fer, les mêmes réducteurs conviennent.

Dans le cas du palladium, les réducteurs peuvent être, en outre, des éléments du milieu réactionnel (phosphine, solvant, oléfine).

Les phosphines sulfonées utilisées dans le procédé selon la présente invention peuvent être préparées en appliquant les procédés connus. Ainsi, conformément à l'enseignement de H. SCHINDLBAUER, Monatsch. Chem. 96, pages 2051-2057 (1965), on peut préparer le sel de sodium de la (p-sulfophényl)diphényl phosphine en faisant réagir, en présence de sodium ou de potassium, du p-chlorobenzène-sulfonate de sodium avec la diphénylchlorophosphine. Selon la méthode décrite dans J. Chem. Soc., pages 276-288 (1958) et dans le brevet anglais n° 1 066 261, on peut préparer des phénylphosphines de formule (I) en faisant appel à la réaction de sulfonation de noyaux aromatiques à l'aide d'oléum, puis en procédant à la neutralisation des groupes sulfoniques formés au moyen d'un dérivé basique approprié d'un des métaux que représente M dans la formule (I). La phosphine sulfonée brute obtenue peut contenir en mélange l'oxyde de phosphine sulfonée correspondant, dont la présence n'est cependant pas gênante pour exécuter le procédé d'isomérisation selon la présente invention.

Le méthyl-2 butène-3 nitrile soumis à I'isomérisation selon le procédé de l'invention peut être mis en oeuvre seul ou en mélange avec d'autres composés.

Ainsi on peut engager du méthyl-2 butène-3 nitrile en mélange avec du méthyl-2 butène-2 nitrile, du pentène-4 nitrile, du pentène-3 nitrile, du pentène-2 nitrile, du butadiène, de l'adiponitrile, du méthyl-2 glutatoronitrile, de l'éthyl-2 succinonitrile ou du valéronitrile.

Ainsi, une variante particulièrement intéressante du procédé de l'invention consiste à traiter le mélange réactionnel provenant de l'hydrocyanation du butadiène par HCN en présence d'une solution aqueuse d'une phosphine sulfonée de formule (I) et d'un composé d'un métal de transition, plus préférentiellement d'un composé du nickel au degré d'oxydation 0, tel que défini précédemment.

Dans le cadre de cette variante préférée, le système catalytique étant déjà présent pour la réaction d'hydrocyanation du butadiène, il suffit d'arrêter toute introduction d'acide cyanhydrique, pour laisser se produire la réaction d'isomérisation.

On peut, le cas échéant, dans cette variante faire un léger balayage du réacteur à l'aide d'un gaz inerte comme l'azote ou l'argon par exemple, afin de chasser l'acide cyanhydrique qui pourrait être encore présent.

La réaction d'isomérisation est généralement réalisée à une température de 10°C à 150°C et de préférence de 60°C à 120°C.

Dans le cas d'une isomérisation suivant immédiatement la réaction d'hydrocyanation du butadiène, il sera avantageux d'opérer à la température à laquelle l'hydrocyanation a été conduite.

La solution catalytique utilisée pour l'isomérisation selon l'invention peut être préparée avant son introduction dans la zone de réaction, par exemple par addition à la solution aqueuse de la phosphine de formule (I), de la quantité appropriée de composé du métal de transition choisi et éventuellement du réducteur. Il est également possible de préparer la solution catalytique "in situ" par simple mélange de ces divers constituants.

La quantité de composé du nickel utilisée est choisie de telle sorte qu'il y ait par litre de solution réactionnelle entre 10⁻⁴ et 1, et de préférence entre 0,005 et 0,5 mole de nickel.

La quantité de phosphine de formule (I) utilisée pour préparer la solution réactionnelle est choisie de telle sorte que le nombre de moles de ce composé rapporté à 1 mole de métal élémentaire soit de 0,5 à 2000 et de préférence de 2 à 300.

Bien que la réaction soit conduite généralement sans tiers-solvant, il peut être avantageux de rajouter un solvant organique inerte, non miscible à l'eau, qui pourra être celui de l'extraction ultérieure.

A titre d'exemples de tels solvants, on peut citer les hydrocarbures aromatiques, aliphatiques ou cycloaliphatiques qui maintiennent à l'état biphasique le milieu réactionnel.

Ainsi dès la fin de la réaction, il est très aisé de séparer, d'une part, une phase aqueuse contenant la phosphine sulfonée de formule (I) et le composé de métal de transition et d'autre part, une phase organique constituée des réactifs engagés dans la réaction des produits de la réaction et le cas échéant du solvant organique non miscible à l'eau.

Parmi les solvants organiques susceptibles d'être utilisés dans le procédé d'isomérisation, on peut citer le benzène, le toluène, les xylènes, l'hexane, le cyclohexane.

Les exemples qui suivent illustrent l'invention.

### EXEMPLE 1

### 1) Préparation de la solution catalytique Ni/TPPTS.

Dans un ballon en verre de 1 litre, muni d'un barreau aimanté et d'un réfrigérant ascendant, on charge 500 cm³ d'une solution de 350 mmol de sel de sodium de triphénylphosphine trisulfonée (TPPTS) dans l'eau; on dégaze cette solution. On introduit ensuite, sous agitation et sous courant d'argon, 19,20 g (70 mmol) de Ni(cyclooctadiène)₂, puis 350 cm³ d'ortho-xylène préalablement dégazé.

On chauffe à 45°C pendant 15 h. Après refroidissement, le système biphasique est décanté et la phase aqueuse fortement colorée en rouge est prélevée.

### 2) Isomérisation du méthyl-2 butène-3 nitrile.

Dans un réacteur en verre de 150cm³, agité à l'aide d'une turbine, on charge

| | |
|---|---|
| - méthyl-2 butène-3 nitrile (2M3BN): | 48,6 g (600 mmol) |
| - solution aqueuse contenant : 0,14 mol/l de Ni et 0,7 mol/l de sel de sodium de la triphénylphosphine trisulfonée (TPPTS): | 16 cm³ (soit 2,24 mmol Ni et 11,2 mmol TPPTS) |

On agite le mélange réactionnel pendant 1,5 h à 90°C

Après refroidissement et décantation, la phase organique finale est dosée par chromatograpie en phase gazeuse (CPG).

On obtient les résultats suivants :

| | |
|---|---|
| Taux de transformation (TT) du 2M3BN: | 95 % |
| Rendement (RT) en pentène-3 nitrile (3PN) par rapport au 2M3BN transformé: | 91,5 % |
| Rendement (RT) en méthyl-2 butène-2 nitrile (2M2BN) par rapport au 2M3BN transformé: | 8 % |

### EXEMPLE 2 - Réduction in situ de Ni II

Dans l'appareillage décrit pour l'exemple 1, on charge :

| | |
|---|---|
| - Ni Cl₂ | 0,75 mmol |
| - TPPTS | 3,15 mmol |
| - NaBH₄ | 0,8 mmol |
| - H₂O | 2,8 g |
| - 2M3BN | 200 mmol |

On fait réagir sous agitation pendant 3 h à 110°C.

On dose la phase organique par CPG.
On obtient les résultats suivants :

| | |
|---|---|
| - TT du 2M3BN | 64 % |
| - RT en 3PN | 97 % |
| - RT en 2M2BN | 3 % |

### EXEMPLE 3

On répète l'exemple 2 avec les charges suivantes :

| | |
|---|---|
| - Ni (Cod)₂ (Cod = cyclooctadiène) | 0,75 mmol |
| - TPPTS | 3,4 mmol |
| - H₂O | 3,8 g |
| - 2M3BN | 200 mmol |
| Température : 90°C Durée : 2 h | |

On obtient les résultats suivants :

| | |
|---|---|
| - TT du 2M3BN | 87 % |
| - RT en 3PN | 93 % |
| - RT en 2M2BN | 2 % |

### EXEMPLE 4

On répète l'exemple 2 avec les charges suivantes :

| | |
|---|---|
| - Ni (Cod)₂ | 0,75 mmol |
| - TPPTS | 3,15 mmol |
| - H₂O | 2,8 g |
| - 2M3BN | 200 mmol |
| Température : 110°C Durée : 0,25 h et 0,5 h | |

On obtient les résultats suivants :

**Tableau 1**

| | Pour 0,25 h | Pour 0,5 h |
|---|---|---|
| TT du 2M3BN | 75 % | 87 % |
| RT en 3PN | 92 % | 94 % |
| RT en 2M2BN | 5 % | 5 % |

### EXEMPLE 5

On répète l'exemple 2 avec les charges suivantes :

| | |
|---|---|
| - Ni (Cod)₂ | 2,5 mmol |
| - TPPTS | 10,8 mmol |
| - H₂O | 5,0 g |
| - 2M3BN | 200 mmol |
| Température : 56°C Durée : 4 h | |

On obtient les résultats suivants :

| | |
|---|---|
| - TT du 2M3BN | 87 % |
| - RT en 3PN | 94 % |

### EXEMPLES 6 A 8

On répète l'exemple 2 avec les charges suivantes :

| | |
|---|---|
| - Ni (Cod)₂ | 0,7 mmol |
| - TPPTS | voir tableau 2 |
| - H₂O | 5 g |
| - 2M3BN | 200 mmol (20 cm³) |
| Température : 90°C Durée : voir tableau 2 | |

Les résultats obtenus sont rassemblés dans le tableau 2 ci-après

**Tableau 2**

| Exemples | TPPTS mmol | Durée | TT du 2M3BN | RT en 3PN | RT en 2M2BN |
|---|---|---|---|---|---|
| Exemple 6 | 2,15 | 0,5 h | 39 % | 80 % | 15 % |
| | | 1,5 h | 81 % | 88 % | 12 % |
| Exemple 7 | 3,6 | 0,5 h | 45 % | 89 % | 9 % |
| | | 1,5 h | 87 % | 92 % | 7 % |
| Exemple 8 | 5,4 | 0,5 h | 49 % | 92 % | 6 % |
| | | 1,5 h | 62 % | 92 % | 6 % |

### EXEMPLES 9 A 10

On répète l'exemple 2 avec les charges suivantes :

| | |
|---|---|
| - Ni (Cod)₂ | voir tableau 3 |
| - TPPTS | 10,5 mmol |
| - H₂O | 15 g |
| - 2M3BN | 600 mmol (60 cm³) |
| Température : 90°C Durée : voir tableau 3 | |

Les résultats obtenus sont rassemblés dans le tableau 3 ci-après.

**Tableau 3**

| Exemples | Ni (Cod)₂ mmol | Durée | TT du 2M3BN | RT en 3PN | RT en 2M2BN |
|---|---|---|---|---|---|
| Exemple 9 | 0,3 | 0,5 h | 11 % | 66 % | 33 % |
| | | 1,5 h | 28 % | 66 % | 33 % |
| Exemple 10 | 2,85 | 0,5 h | 48 % | 93 % | 6 % |
| | | 1,5 h | 94 % | 93 % | 6 % |

### EXEMPLE 11

### Essais de recyclage

On répète l'exemple 2, mais après l'essai la phase organique est séparée et dosée par CPG, tandis que la phase aqueuse contenant le catalyseur au nickel et la TPPTS est recyclée avec une nouvelle charge de 2M3BN.

On charge les composés suivants :

| | |
|---|---|
| - Ni (Cod)₂ | 0,74 mmol |
| - TPPTS | 3,4 mmol |
| - H₂O | 3,8 g |
| - 2M3BN | voir tableau 4 |
| Température : 90°C Durée : 90 min pour chaque essai | |

Les résultats obtenus sont rassemblés dans le tableau 4 ci-après

**Tableau 4**

| Recyclage | 2M3BN chargé (mmol) | TT du 2M3BN | RT en 3PN | RT en 2M2BN |
|---|---|---|---|---|
| 0 | 211 | 93 % | 92 % | 5 % |
| 1 | 205 | 78 % | 96 % | 4 % |
| 2 | 225 | 60 % | 90 % | 5 % |

### EXEMPLE 12

### Essais de recyclage

On répète l'exemple 11, avec les charges suivantes :

| | |
|---|---|
| - Ni (Cod)₂ | 2,26 mmol |
| - TPPTS | 10,1 mmol |
| - H₂O | 16 g |
| - 2M3BN | voir tableau 5 |
| Température : 90°C Durée : voir tableau 5 | |

Les résultats obtenus sont rassemblés dans le tableau 5 ci-après

**Tableau 5**

| Recyclage | 2M3BN chargé (mmol) | Durée (min) | TT du 2M3BN | RT en 3PN | RT en 2M2BN |
|---|---|---|---|---|---|
| 0 | 600 | 90 | 95 % | 91 % | 8 % |
| 1 | 600 | 90 | 73 % | 90 % | 9 % |
| 2 | 600 | 90 | 47 % | 90 % | 9 % |
| | | 180 | 72 % | 90 % | 9 % |

## Revendications

1. Procédé d'isomération du méthyl-2 butène-3 nitrile en un pentène-nitrile linéaire, caractérisé en ce que la réaction est effectuée en présence d'une solution aqueuse :
- d'au moins une phosphine sulfonée de formule générale (I) 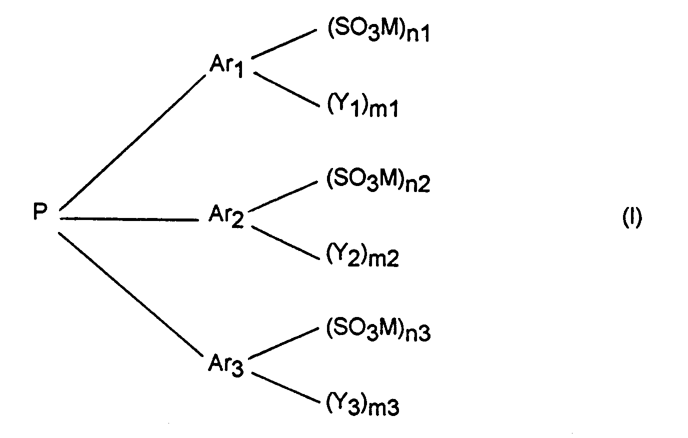 dans laquelle :
* Ar₁, Ar₂ et Ar₃, identiques ou différents, représentent des groupements aryles
* Y₁, Y₂ et Y₃, identiques ou différents, représentent
. un radical alkyle ayant 1 à 4 atomes de carbone,
. un radical alcoxy ayant 1 à 4 atomes de carbone,
. un atome d'halogène,
. un radical CN,
. un radical NO₂
. un radical OH,
. un radical NR₁R₂, dans la formule duquel R₁ et R₂, identiques ou différents, représentent un radical alkyle ayant 1 à 4 atomes de carbone,
* M est un reste cationique minéral ou organique choisi, de manière à ce que le composé de formule (I) soit soluble dans l'eau, dans le groupe comprenant :
. H⁺,
. les cations dérivés des métaux alcalins ou alcalino-terreux,
. N(R₃R₄R₅R₆)⁺ avec R₃,R₄,R₅ et R₆, identiques ou différents, représentant un radical alkyle ayant 1 à 4 atomes de carbone ou un atome d'hydrogène,
. les autres cations dérivés des métaux dont les sels de l'acide benzènesulfonique sont solubles dans l'eau,
* m₁, m₂ et m₃ sont des nombres entiers, identiques ou différents, de 0 à 5,
* n₁, n₂ et n₃ sont des nombres entiers, identiques ou différents, de 0 à 3, l'un d'entre eux au moins étant égal ou supérieur à 1
- et d'au moins un composé d'un métal de transition.

2. Procédé selon la revendication 1, caractérisé en ce que la phosphine sulfonée utilisée est choisie parmi les phosphines de formule (I), dans laquelle :
- Ar₁, Ar₂ et Ar₃ sont des groupements phényle,
- Y₁, Y₂ et Y₃ représentent des groupements choisis parmi
. les radicaux alkyle ayant de 1 à 2 atomes de carbone,
. les radicaux alkoxy ayant de 1 à 2 atomes de carbone,
- M représente un cation choisi parmi le groupe comprenant
. H⁺
. les cations dérivés de Na, K, Ca, Ba
. NH₄+
. les cations tétraméthylammonium, tétraéthylammonium, tétrapropylammonium, tétrabutylammonium
- m₁, m₂ et m₃ sont des nombres entiers de 0 à 3
- n₁, n₂ et n₃ sont des nombres entiers de 0 à 3, l'un au moins étant également supérieur à 1.

3. Procédé selon l'une des revendications 1 ou 2, caractérisé en ce que la phosphine sulfonée utilisée est choisie parmi les sels de sodium, de potassium, de calcium, de baryum, d'ammonium, de tétraméthylammonium et de tétraéthylammonium, des mono(sulfophényl) diphényl-phosphine, di(sulfophényl) phényl-phosphine et tri(sulfophényl)-phosphine dans les formules desquelles les groupements SO₃ sont de préférence en position méta.

4. Procédé selon l'une des revendications 1 à 3, caractérisé en ce que l'on utilise comme composé de métal de transition les composés du nickel, du palladium ou du fer, solubles dans l'eau ou capables de passer en solution dans les conditions de la réaction et de préférence les composés du nickel.

5. Procédé selon la revendication 4, caractérisé en ce que le composé du nickel utilisé est un composé de nickel zéro.

6. Procédé selon la revendication 4, caractérisé en ce que le composé du nickel utilisé est un composé de nickel à un degré d'oxydation supérieur à zéro.

7. Procédé selon la revendication 6, caractérisé en ce que l'on ajoute au milieu réactionnel un réducteur du nickel, organique ou minéral, réagissant préférentiellement avec le nickel dans les conditions réactionnelles.

8. Procédé selon la revendication 7, caractérisé en ce que le réducteur est ajouté en quantité telle que le nombre d'équivalents d'oxydo-réduction est compris entre 1 et 10.

9. Procédé selon l'une des revendications 1 à 8, caractérisé en ce que le méthyl-2 butène-3 nitrile soumis à l'isomérisation est mis en oeuvre seul ou en mélange avec d'autres composés comme le méthyl-2 butène-2 nitrile, le pentène-4 nitrilile, le pentène-3 nitrile, le pentène-2 nitrile, le butadiène, l'adiponitrile, le méthyl-2 glutaronitrile, l'éthyl-2 succinonitrile et le valéronitrile.

10. Procédé selon l'une des revendications 1 à 9, caractérisé en ce que l'on traite le mélange réactionnel provenant de l'hydrocyanation du butadiène par HCN en présence d'une solution aqueuse d'une phosphine sulfonée de formule (I) et d'un composé d'un métal de transition, plus préférentiellement d'un composé du nickel au degré d'oxydation 0.

11. Procédé selon l'une des revendications 1 à 10, caractérisé en ce que la réaction d'isomérisation est réalisée à une température de 10°C à 150°C et de préférence de 60°C à 120°C.

12. Procédé selon l'une des revendications 4 à 11, caractérisé en ce que la quantité de composé du nickel utilisée est choisie de telle sorte qu'il y ait par litre de solution réactionnelle entre 10⁻⁴ et 1, et de préférence entre 0,005 et 0,5 mole de nickel.

13. Procédé selon l'une des revendications 1 à 12, caractérisé en ce que la quantité de phosphine de formule (I) utilisée pour préparer la solution réactionnelle est choisie de telle sorte que le nombre de moles de ce composé rapporté à 1 mole de métal élémentaire soit de 0,5 à 2000 et de préférence de 2 à 300.

## Claims

1. Process for the isomerization of 2-methyl-3-butenenitrile into a linear pentenenitrile, characterized in that the reaction is carried out in the presence of an aqueous solution:
- of at least one sulphonated phosphine of general formula (I) 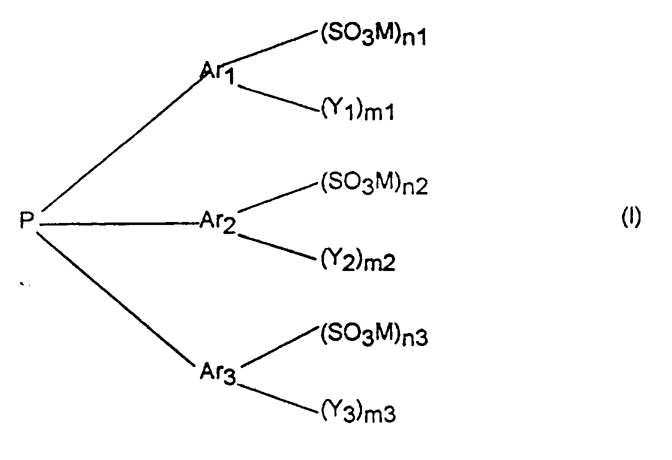 in which:
* Ar₁, Ar₂ and Ar₃, which may be identical or different, represent aryl groups
* Y₁, Y₂ and Y₃, which may be identical or different, represent
. an alkyl radical having 1 to 4 carbon atoms,
. an alkoxy radical having 1 to 4 carbon atoms,
. a halogen atom,
. a CN radical,
. an NO₂ radical
. an OH radical,
. a radical NR₁R₂, in which formula R₁ and R₂, which may be identical or different, represent an alkyl radical having 1 to 4 carbon atoms,
* M is an inorganic or organic cationic residue chosen, so that the compound of formula (I) is water-soluble, from the group comprising:
. H⁺,
. cations derived from alkali metals or alkaline-earth metals,
. N(R₃R₄R₅R₆)⁺ with R₃, R₄, R₅ and R₆, which may be identical or different, representing an alkyl radical having 1 to 4 carbon atoms or a hydrogen atom,
. other cations derived from metals for which the benzenesulphonic acid salts are water-soluble,
* m₁, m₂ and m₃ are integers, which may be identical or different, from 0 to 5,
* n₁, n₂ and n₃ are integers, which may be identical or different, from 0 to 3, at least one among these being equal to or greater than 1
- and of at least one transition metal compound.

2. Process according to Claim 1, characterized in that the sulphonated phosphine used is chosen from the phosphines of formula (I) in which:
- Ar₁, Ar₂ and Ar₃ are phenyl groups,
- Y₁, Y₂ and Y₃ represent groups chosen from
. alkyl radicals having from 1 to 2 carbon atoms,
. alkoxy radicals having from 1 to 2 carbon atoms,
- M represents a cation chosen from the group comprising
. H⁺
. cations derived from Na, K, Ca and Ba
. NH₄⁺
. tetramethylammonium, tetraethylammonium, tetrapropylammonium and tetrabutylammonium cations
- m₁, m₂ and m₃ are integers from 0 to 3
- n₁, n₂ and n₃ are integers from 0 to 3, at least one also being greater than 1.

3. Process according to either of Claims 1 and 2, characterized in that the sulphonated phosphine used is chosen from the sodium, potassium, calcium, barium, ammonium, tetramethylammonium and tetraethylammonium salts of mono(sulphophenyl)diphenylphosphine, di(sulphophenyl)phenylphosphine and tri(sulphophenyl)phosphine, in the formulae in which the SO₃ groups are preferably in the meta-position, are more particularly preferred.

4. Process according to one of Claims 1 to 3, characterized in that compounds of nickel, of palladium or of iron, which are water-soluble or capable of dissolving under the reaction conditions, and preferably nickel compounds, are used as transition metal compound.

5. Process according to Claim 4, characterized in that the nickel compound used is a nickel zero compound.

6. Process according to Claim 4, characterized in that the nickel compound used is a compound of nickel with an oxidation state greater than zero.

7. Process according to Claim 6, characterized in that an organic or inorganic nickel-reducing agent which reacts preferentially with nickel under the reaction conditions is added to the reaction medium.

8. Process according to Claim 7, characterized in that the reducing agent is added in an amount such that the number of redox equivalents is between 1 and 10.

9. Process according to one of Claims 1 to 8, characterized in that the 2-methyl-3-butenenitrile which undergoes the isomerization is used alone or as a mixture with other compounds, for instance 2-methyl-2-butenenitrile, 4-pentenenitrile, 3-pentenenitrile, 2-pentenenitrile, butadiene, adiponitrile, 2-methylglutaronitrile, 2-ethylsuccinonitrile or valeronitrile.

10. Process according to one of Claims 1 to 9, characterized in that the reaction mixture obtained from the hydrocyanation of butadiene with HCN is treated in the presence of an aqueous solution of a sulphonated phosphine of formula (I) and of a transition metal compound, more preferably a compound of nickel of oxidation state 0.

11. Process according to one of Claims 1 to 10, characterized in that the isomerization reaction is performed at a temperature of 10°C to 150°C and preferably of 60°C to 120°C.

12. Process according to one of Claims 4 to 11, characterized in that the amount of nickel compound used is chosen such that there is between 10⁻⁴ and 1 and preferably between 0.005 and 0.5 mol of nickel per litre of reaction solution.

13. Process according to one of Claims 1 to 12, characterized in that the amount of phosphine of formula (I) used in order to prepare the reaction solution is chosen such that the number of moles of this compound relative to 1 mol of elemental metal is from 0.5 to 2000 and preferably from 2 to 300.

## Patentansprüche

1. Verfahren zur Isomerisierung Von 2-Methyl-3-butennitril zu einem geradkettigen Pentennitril, dadurch gekennzeichnet, daß die Reaktion in Gegenwart einer wässerigen Lösung durchgeführt wird, die enthält:
- mindestens ein sulfoniertes Phosphin der allgemeinen Formel (I): 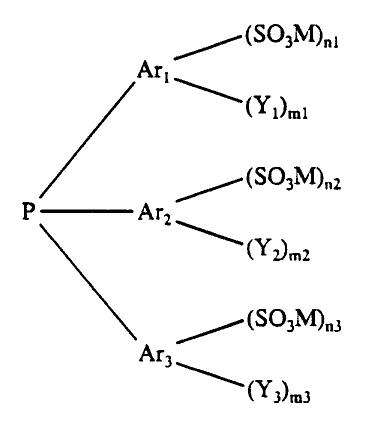 in der:
• Ar₁, Ar₂ und Ar₃, die gleich oder verschieden sein können, Arylgruppen darstellen,
• Y₁, Y₂ und Y₃, die gleich oder verschieden sein können,
- einen Alkylrest mit 1 bis 4 Kohlenstoffatomen,
- einen Alkoxyrest mit 1 bis 4 Kohlenstoffatomen,
- ein Halogenatom,
- einen CN-Rest,
- einen NO₂-Rest,
- einen OH-Rest
oder
- einen Rest NR₁R₂ darstellen, wobei in der Formel dieses Rests R₁ und R₂, die gleich oder verschieden sein können, einen Alkylrest mit 1 bis 4 Kohlenstoffatomen bezeichnen,
• M ein anorganischer oder organischer kationischer Rest ist, der mit der Maßgabe, daß die Verbindung der Formel (I) wasserlöslich sein muß, unter folgenden Resten ausgewählt ist:
- H⁺,
- von Alkali- oder Erdalkalimetallen abgeleiteten Kationen,
- N(R₃R₄R₅R₆)⁺, wobei R₃, R₄, R₅ und R₆, die gleich oder verschieden sein können, einen Alkylrest mit 1 bis 4 Kohlenstoffatomen oder ein Wasserstoffatom darstellen,
und
- anderen, von Metallen abgeleiteten Kationen, deren Benzolsulfonsäuresalze wasserlöslich sind,
• m1, m2 und m3 gleiche oder verschiedene ganze Zahlen von 0 bis 5 sind und
• n1, n2 und n3 gleiche oder verschiedene ganze Zahlen von 0 bis 3 sind, wobei zumindest eine dieser Zahlen n1, n2 und n3 größer oder gleich 1 ist,
und
- mindestens eine Übergangsmetallverbindung.

2. Verfahren nach Anspruch 1, dadurch gekennzeichnet, daß das verwendete sulfonierte Phosphin unter den Phosphinen der Formel (I) ausgewählt ist, wobei in dieser Formel (I):
- Ar₁, Ar₂ und Ar₃ Phenylgruppen darstellen,
- Y₁, Y₂ und Y₃ Gruppen darstellen, die ausgewählt sind unter:
• den Alkylresten mit 1 bis 2 Kohlenstoffatomen
und
• den Alkoxyresten mit 1 bis 2 Kohlenstoffatomen,
- M ein Kation darstellt, das unter folgenden Kationen ausgewählt ist:
• H⁺,
• den von Na, K, Ca und Ba abgeleiteten Kationen,
• NH₄⁺
und
• den Tetramethyl-, Tetraethyl-, Tetrapropyl- und Tetrabutylammoniumkationen,
- m1, m2 und m3 ganze Zahlen von 0 bis 3 sind
und
- n1, n2 und n3 ganze Zahlen von 0 bis 3 sind, wobei ebenfalls zumindest eine dieser Zahlen n1, n2 und n3 größer als 1 ist.

3. Verfahren nach einem der Ansprüche 1 oder 2, dadurch gekennzeichnet, daß das verwendete sulfonierte Phosphin unter den Natrium-, Kalium-, Calcium-, Barium-, Ammonium-, Tetramethylammonium- und Tetraethylammoniumsalzen des Mono(sulfophenyl)-diphenylphosphins, des Di-(sulfophenyl)-phenylphosphins und des Tri(sulfophenyl)-phosphins ausgewählt ist, wobei sich die SO₃-Gruppen dieser Verbindungen bevorzugt in meta-Stellung befinden.

4. Verfahren nach einem der Ansprüche 1 bis 3, dadurch gekennzeichnet, daß als Übergangsmetallverbindung eine Nickel-, Palladium- oder Eisenverbindung, die wasserlöslich ist oder die unter den Reaktionsbedingungen in Lösung gebracht werden kann, und vorzugsweise eine Nickelverbindung verwendet wird.

5. Verfahren nach Anspruch 4, dadurch gekennzeichnet, daß die verwendete Nickelverbindung eine Nickel(0)-Verbindung ist.

6. Verfahren nach Anspruch 4, dadurch gekennzeichnet, daß die Oxidationsstufe des Nickels in der verwendeten Nickelverbindung größer Null ist.

7. Verfahren nach Anspruch 6, dadurch gekennzeichnet, daß dem Reaktionsmedium ein organisches oder anorganisches Reduktionsmittel des Nickels zugegeben wird, das unter den Reaktionsbedingungen bevorzugt mit dem Nickel reagiert.

8. Verfahren nach Anspruch 7, dadurch gekennzeichnet, daß das Reduktionsmittel in einer solchen Menge zugegeben wird, daß die Anzahl der Redox-Äquivalente im Bereich von 1 bis 10 liegt.

9. Verfahren nach einem der Ansprüche 1 bis 8, dadurch gekennzeichnet, daß das der Isomerisierung unterworfene 2-Methyl-3-butennitril als Einzelsubstanz oder in Form eines Gemischs mit anderen Verbindungen, wie 2-Methyl-2-butennitril, 4-Pentennitril, 3-Pentennitril, 2-Pentennitril, Butadien, Adipinsäuredinitril, 2-Methylglutarsäuredinitril, 2-Ethylbernsteinsäuredinitril und Valeronitril, eingesetzt wird.

10. Verfahren nach einem der Ansprüche 1 bis 9, dadurch gekennzeichnet, daß das aus der Hydrocyanierungsreaktion des Butadiens mit HCN stammende Reaktionsgemisch mit einer wässerigen Lösung eines sulfonierten Phosphins der Formel (I) und einer Übergangsmetallverbindung (vorzugsweise einer Nickel(0)-Verbindung) behandelt wird.

11. Verfahren nach einem der Ansprüche 1 bis 10, dadurch gekennzeichnet, daß die Isomerisierungsreaktion bei einer Temperatur von 10 bis 150 °C und vorzugsweise von 60 bis 120 °C durchgeführt wird.

12. Verfahren nach einem der Ansprüche 4 bis 11, dadurch gekennzeichnet, daß die verwendete Menge der Nickelverbindung so gewählt wird, daß die Konzentration des Nickels in der Realationslösung 10⁻⁴ bis 1 mol/l und vorzugsweise 0,005 bis 0,5 mol/l beträgt.

13. Verfahren nach einem der Ansprüche 1 bis 12, dadurch gekennzeichnet, daß die zur Herstellung der Reaktionslösung verwendete Menge des Phosphins der Formel (I) so gewählt wird, daß die Anzahl der Mole dieser Verbindung, bezogen auf 1 Mol des elementaren Metalls, 0,5 bis 2000 und vorzugsweise 2 bis 300 beträgt.
